# DEMANDE DE BREVET EUROPEEN

(11) **EP 4 678 211 A1**
(43) Date de publication de la demande: **14.01.2026**
(21) Numéro de dépôt: 25176703.4
(22) Date de dépôt: 15.05.2025
(51) Int. Cl.: A61M 16/00, A61M 16/08, A61M 16/06, A61M 16/10

(54) **INSTALLATION DE FOURNITURE DE GAZ THÉRAPEUTIQUE**

(30) Priorité: 12.07.2024 FR 2407674
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, 75007 Paris (FR); PATEL, Akshar, 75007 Paris (FR)
(74) Mandataire: Air Liquide

(57) **Abrégé**

L'invention concerne une installation de fourniture de gaz respiratoire (50) à un patient (P) comprenant un appareil de délivrance de gaz (1), un masque respiratoire (10), une conduite d'alimentation en gaz (13) pour acheminer le gaz respiratoire provenant de l'appareil de délivrance de gaz (1) jusqu'au masque (10), et une ligne d'amenée de pression (16) pour amener la pression de gaz du masque (10) à l'appareil de délivrance de gaz (1). Un module de filtration de gaz (40) comprenant au moins un filtre, est agencé entre l'appareil de délivrance de gaz (1) et la conduite d'alimentation en gaz (13). Un dispositif de raccordement (2) met en communication fluidique la ligne d'amenée de pression (16) et l'appareil de délivrance de gaz (1), et des moyens de fixation (24, 25) solidarisent le dispositif de raccordement (2) au module de filtration de gaz (131).

## Description

L'invention concerne une installation de fourniture de gaz thérapeutique intégrant un appareil de délivrance de gaz pouvant être utilisés pour fournir un gaz thérapeutique (i.e. gaz pur ou mélange gazeux) à un patient conscient dans différents lieux de soins, en particulier à l'hôpital, y compris dans le cadre d'inhalations de longue durée, par exemple de plusieurs heures, tout en minimisant les pertes de gaz.

Certaines thérapies nécessitent l'administration à des personnes, i. des patients, de gaz thérapeutiques formés d'un mélange de plusieurs constituants gazeux, par exemple protoxyde d'azote (N₂O) et oxygène (O₂) pour amoindrir les états d'anxiété, produire un effet sédatif et/ou atténuer la douleur aiguë, ou un mélange d'argon (Aᵣ) et d'oxygène (O₂) en cas de thrombectomie mécanique suite à un accident vasculaire cérébral (AVC).

Parfois, des inhalations de longues durées sont nécessaires, typiquement d'une à plusieurs heures. Dans ce cas, une délivrance de gaz intermittente, c'est-à-dire non-continue, est donc souvent préférée.

Ainsi, EP3981454 décrit un appareil de délivrance, i.e. fourniture, de gaz à un patient permettant de limiter la consommation de gaz, lequel fonctionne comme une valve à la demande, tout en assurant un effort inspiratoire bas du patient pour garantir son confort respiratoire. L'appareil de délivrance est raccordé à une conduite de gaz (e.g. tuyau flexible) alimentant un masque respiratoire. Une conduite d'amenée de pression (e.g. tuyau flexible) relie par ailleurs une prise de pression du masque à un capteur de pression qui est disposé dans l'appareil. Les mesures de pression permettant d'améliorer la délivrance du gaz thérapeutique.

Etant donné que l'environnement hospitalier où se trouve le patient est souvent encombré de dispositifs médicaux, les longueurs de la conduite d'alimentation en gaz et de celle de la conduite ou ligne d'amenée de pression sont généralement de plusieurs mètres, par exemple d'au moins 4 mètres environ. Or, utiliser telles longueurs engendre un risque de déconnexion/débranchement de ces conduites, qui ne sont pas forcément détectées immédiatement par le personnel soignant.

Ainsi, en cas de débranchement de la conduite d'alimentation en gaz au niveau de son raccordement à l'appareil de délivrance, alors que la ligne d'amenée de pression reste correctement branchée, l'appareil ne peut détecter un dysfonctionnement, c'est-à-dire la rupture d'alimentation en gaz, car la pression continue à lui être fournie par la ligne d'amenée de pression. On comprend aisément qu'un tel débranchement inopiné de la conduite d'alimentation en gaz peut s'avérer dangereux pour le patient, s'il n'est pas détecté rapidement, car le patient ne reçoit plus le gaz thérapeutique nécessaire à son traitement.

Un problème est dès lors de pouvoir améliorer un appareil de fourniture de gaz du type de celui décrit par EP3981454, pour permettre de détecter rapidement toute rupture de l'alimentation en gaz et/ou d'amenée de pression.

Une solution repose alors sur une installation de fourniture de gaz respiratoire à un patient, c'est-à-dire une personne, comprenant :
- un appareil de délivrance de gaz comprenant un port de sortie de gaz pour fournir un gaz respiratoire et un port de mesure de pression,
- un masque respiratoire comprenant une entrée de gaz et une sortie de pression,
- une conduite (i.e. ligne) d'alimentation en gaz pour acheminer le gaz respiratoire provenant du port de sortie de gaz de l'appareil de délivrance de gaz jusqu'à l'entrée de gaz du masque, et
- une ligne (ou conduit) d'amenée de pression pour amener la pression de gaz de la sortie de pression du masque au port de mesure de pression de l'appareil de délivrance de gaz.

De plus, l'installation de fourniture de gaz selon l'invention comprend en outre :
- un module de filtration de gaz comprenant au moins un filtre, agencé entre le port de sortie de gaz de l'appareil de délivrance de gaz et la conduite d'alimentation en gaz de sorte que le gaz provenant du port de sortie de gaz traverse ledit module de filtration de gaz avant d'entrer dans la conduite d'alimentation en gaz.
- un dispositif de raccordement comprenant un corps principal traversé par un passage interne mettant en communication fluidique la ligne d'amenée de pression avec le port de mesure de pression de l'appareil de délivrance de gaz, et des moyens de fixation pour permettre une solidarisation du dispositif de raccordement au module de filtration de gaz.

Selon le mode de réalisation considéré, l'installation de fourniture de gaz de l'invention peut comprendre l'une ou plusieurs des caractéristiques suivantes :
- le corps principal du dispositif de raccordement est en un matériau souple, c'est-à-dire qu'il est flexible.
- le corps principal est en un matériau souple présentant une dureté Shore supérieure ou égale à 60.
- le matériau souple est choisi parmi les matériaux silicone.
- le passage interne du corps principal comprend une entrée de passage et une sortie de passage, c'est-à-dire des orifices d'entrée et de sortie communiquant fluidiquement avec le passage de gaz, i.e. son lumen.
- le passage interne du corps principal comprend ou est un canal, un conduit ou analogue.
- le corps principal comprend un connecteur amont, portant l'entrée de passage, configuré pour permettre un raccordement fluidique de la ligne (i.e. conduit, tuyau ou analogue) d'amenée de pression
- le corps principal comprend par ailleurs un connecteur aval, portant la sortie de passage, configuré pour permettre un raccordement fluidique au port de mesure de pression de l'appareil de délivrance de gaz.
- le corps principal du dispositif de raccordement comprend deux expansions, agencées face à face et espacées l'une de l'autre, en particulier agencées sensiblement parallèles l'une à l'autre, notamment elles sont symétriques l'une par rapport à l'autre.
- les expansions forment des bras, des ailes ou analogues.
- les expansions portent les moyens de fixation.
- les expansions comprennent chacune une extrémité proximale solidaire du corps principal.
- les expansions comprennent chacune une extrémité distale libre.
- les moyens de fixation sont agencées au niveau des extrémités distales libres des expansions.
- les expansions se projettent en éloignement par rapport au corps principal.
- les expansions sont formées d'une seule pièce avec le corps principal, par exemple par injection-moulage ou analogue.
- le module de filtration comprend un corps de module comprenant un compartiment interne dans lequel est logé au moins un filtre.
- le module de filtration comprend un conduit amont et un conduit aval, telles des tubulures, connecteurs ou analogues, agencés de part et d'autre du corps de module, lesdits conduit amont et conduit aval étant en communication fluidique avec le compartiment interne.
- le conduit amont (e.g. connecteur amont) du module de filtration est configuré pour permettre un raccordement au port de sortie de gaz de l'appareil de délivrance de gaz.
- le conduit aval (e.g. connecteur aval) du module de filtration est configuré pour permettre un raccordement de la conduite d'alimentation en gaz alimentant le masque.
- le raccordement au port de sortie de gaz et/ou au conduit de gaz se fait par emboitement ou analogue.
- les moyens de fixation comprennent des ouvertures de fixation configurées pour loger les conduits amont et aval du module de filtration de manière à assurer une solidarisation ou un maintien du dispositif de raccordement, en particulier de son corps principal, couplé au module de filtration.
- les ouvertures de fixation sont aménagées au niveau des extrémités distales libres des expansions, en particulier au travers de la paroi desdites expansions.
- les conduits amont et aval du module de filtration sont insérés au sein des ouvertures de fixation des moyens de fixation par déformation élastique des expansions du dispositif de raccordement.
- les conduits amont et aval du module de filtration sont tubulaires, préférentiellement sensiblement cylindriques.
- le corps de module est en polymère de type polycarbonate ou analogue.
- le module de filtration de gaz est pris en sandwich entre les deux expansions et maintenu par les moyens de fixation portés par lesdites deux expansions.
- le corps principal du dispositif de raccordement a une forme générale en U.
- le module de filtration de gaz comprend au moins un filtre bactériologique.
- la ligne d'amenée de pression, aussi appelé « conduit d'amenée de pression », constitue une liaison pneumatique entre le masque respiratoire et le capteur de pression de l'appareil de délivrance de gaz, permettant d'acheminer la pression gazeuse depuis le masque jusqu'au capteur de pression de l'appareil de délivrance de gaz.
- la ligne d'amenée de pression a une longueur d'au moins 4 m, typiquement de 3 à 5 m environ, et/ou un diamètre de 2,5 à 5 mm environ.
- la conduite d'alimentation en gaz a une longueur d'au moins 4 m, typiquement de 3 à 5 m environ et/ou un diamètre de 20 à 25 mm environ.
- la ligne d'amenée de pression et/ou la conduite d'alimentation en gaz comprennent des tuyaux flexibles en polymère ou en silicone.
- l'appareil de délivrance de gaz comprend un capteur de pression interne configuré pour déterminer la pression du gaz amené par la ligne d'amenée de pression.
- l'appareil de délivrance de gaz comprend des moyens d'alarme ou d'alerte visuelle et/ou auditive permettant d'alerter l'utilisateur en cas de débranchement.

Selon le mode de réalisation considéré, l'appareil de délivrance de gaz de l'installation selon l'invention peut comprendre l'une ou plusieurs des caractéristiques suivantes :
- il comprend un circuit de gaz interne en communication fluidique avec un dispositif à vanne agencée sur le circuit de gaz interne pour contrôler le débit de gaz thérapeutique circulant dans le circuit de gaz interne.
- il comprend une unité de contrôle à microprocesseur pilotant le dispositif à vanne pour fixer ou ajuster le débit de gaz traversant ledit dispositif à vanne
- il comprend un capteur de pression configuré pour déterminer (i.e. mesurer) la pression de gaz fournie par la ligne d'amenée de pression, c'est-à-dire la pression au masque, et fournir à l'unité de contrôle une ou des mesures de pression de gaz.
- l'unité de contrôle est configurée pour comparer la ou les mesures de pression de gaz fournies par le capteur de pression à une valeur-seuil de pression donnée et commander le dispositif à vanne pour ajuster le débit de gaz en fonction de ladite comparaison.
- lorsque l'unité de contrôle détermine que la pression de gaz mesurée dans le masque est inférieure ou égale à la valeur-seuil de pression donnée, ladite unité de contrôle est configurée pour commander le dispositif à vanne pour augmenter le débit de gaz thérapeutique traversant ledit dispositif à vanne et alimentant le circuit de gaz interne.
- la valeur-seuil de pression est inférieure ou égale à 0 mbar.
- la valeur-seuil de pression est inférieure ou égale à -0.25 mbar, de préférence inférieure ou égale à -0.5 mbar.
- la valeur-seuil de pression est mémorisée au sein de l'unité de contrôle.
- la valeur-seuil de pression est mémorisée par le microprocesseur ou par une mémoire de stockage de données.
- la valeur-seuil de pression est réglable.
- le passage de gaz interne de l'appareil comprend un ou plusieurs conduits, tuyaux ou analogue.
- le capteur de pression comprend un capteur de pression différentiel.
- le capteur de pression est relié électriquement à l'unité de contrôle.
- le dispositif à vanne comprend une vanne proportionnelle.
- le capteur de pression est configuré pour fournir à l'unité de contrôle une ou des mesures de pression de gaz, préférentiellement plusieurs mesures de pression successives, sous forme de valeurs numériques ou de signaux représentatifs de telles valeurs numériques (par ex. des signaux de tensions, lesquels valeurs ou signaux peuvent être traités tels quels ou convertis en valeurs numériques par l'unité de contrôle.
- une source d'alimentation électrique de type cordon et prise d'alimentation au secteur (e.g. 110/220 V) et/ou une batterie interne, de préférence rechargeable, alimente en courant électrique tous les composants de l'appareil qui nécessitent de la puissance électrique pour fonctionner, par exemple l'unité de contrôle, un écran d'affichage, une ou des LED, un dispositif d'alarme sonore et/ou visuelle...
- l'unité de contrôle, au moins une partie du passage de gaz interne, le capteur de pression et/ou le dispositif à vanne sont agencés dans le boitier.
- le capteur de pression est configuré ou contrôlé pour déterminer la pression gazeuse à des intervalles de temps donnés, de préférence toutes les 20 msec ou moins, préférentiellement toutes les 10 msec ou moins, voire toutes les 5 msec ou moins.
- l'unité de contrôle est configurée pour piloter le dispositif à vanne, en particulier la vanne proportionnelle, pour ajuster (i.e. fixer ou modifier) le débit de gaz traversant ledit dispositif à vanne en fonction de la comparaison faite par l'unité de contrôle entre la pression mesurée au masque et la valeur-seuil de pression donnée préfixée servant de pression référence, en particulier pour augmenter le débit de gaz thérapeutique traversant le dispositif à vanne lorsque l'unité de contrôle détermine que la pression de gaz mesurée dans le masque est inférieure ou égale à la valeur-seuil de pression donnée, où la pression seuil est inférieure ou égale à 0 mbar, de préférence inférieure ou égale à -0.25 mbar
- l'unité de contrôle à microprocesseur comprend un ou plusieurs microprocesseurs, de préférence un (ou des) microcontrôleur.
- le (ou les) microprocesseur(s) met en œuvre un ou plusieurs algorithmes.
- l'unité de contrôle comprend une ou plusieurs mémoires de stockage de données ou autres, par exemple des tables de référence.
- l'unité de contrôle à microprocesseur comprend une carte électronique portant le ou les microprocesseurs, de préférence un ou des microcontrôleurs.
- l'appareil de délivrance de gaz comprend en outre un débitmètre ou capteur de débit agencé dans le passage de gaz interne pour mesurer le débit de gaz circulant dans ledit passage de gaz interne.
- le capteur de débit (i.e. débitmètre) agencé dans le passage de gaz interne, en aval du dispositif à vanne, en particulier la vanne proportionnelle, de manière à pouvoir mesurer le débit de gaz fourni par ledit dispositif à vanne.
- le capteur de débit est relié électriquement à l'unité de contrôle et lui fournissent les mesures qu'ils opèrent.
- le capteur de débit est un capteur de débit massique ou un capteur de pression différentielle.
- l'appareil comprend en outre une (ou des) valve unidirectionnelle agencée dans le passage de gaz interne, en particulier en aval du réservoir déformable.
- l'appareil comprend en outre une interface homme-machine (IHM) comprenant un écran d'affichage d'informations, de préférence un écran tactile, et/ou une ou des touches ou boutons de sélection, notamment des touches virtuelles s'affichant sur l'écran tactile, et/ou un dispositif de mise en route, tel un bouton allumé/éteint ou « on/off », et/ou d'autres éléments.
- l'appareil comprend en outre des moyens (i.e. un système) d'alarme pour alerter l'utilisateur en cas de problème affectant l'appareil ou le gaz, par exemple un défaut de vanne ou de capteur, une mauvaise composition gazeuse (e.g. mélange hypoxique) ou autres.
- le système d'alarme peut comprendre des moyens ou un dispositif d'émission de signaux sonores et/ou visuels.
- l'appareil comprend en outre des moyens, i.e. un système, d'alarme de débranchement pour alerter l'utilisateur d'une déconnexion du corps principal du dispositif de raccordement, en particulier à partir de la valeur de pression mesurée au masque, notamment en l'analysant et/ou en la comparant à une valeur de pression-seuil.
- une déconnexion (i.e. débranchement) du corps principal du dispositif de raccordement est détectée lorsque la valeur de pression mesurée au masque reste inférieure à un seuil de débranchement donné, pendant une durée-seuil déterminée.
- le seuil de débranchement est une pression de l'ordre de 0 mb relatifs, par exemple comprise entre -0.25mb et +0.25mb relatifs.
- la durée-seuil est de l'ordre de quelques secondes à quelques dizaines de secondes, par exemple de l'ordre de 5 à 30 secondes environ.
- l'unité de contrôle pilote les moyens d'alarme et/ou les moyens d'alarme de débranchement.

Selon le mode de réalisation considéré, l'installation de fourniture de gaz thérapeutique selon l'invention peut comprendre en outre l'une ou plusieurs des caractéristiques additionnelles suivantes :
- le masque respiratoire est un masque facial (i.e. naso-buccal) couvrant le nez et la bouche du patient, en utilisation, c'est-à-dire lorsqu'il est porté par ledit patient.
- elle comprend en outre une source de gaz thérapeutique raccordée fluidiquement au passage ou circuit interne de gaz de l'appareil de délivrance de gaz pour alimenter ledit passage de gaz en gaz thérapeutique.
- la source de gaz thérapeutique comprend un ou plusieurs récipients de gaz, notamment des bouteilles.
- la source de gaz thérapeutique comprend un récipient de gaz contenant un mélange gazeux O₂/N₂O, de préférence un mélange équimolaire de O₂/N₂O (i.e. 50 mol.%/50 mol.%).
- alternativement, la source de gaz thérapeutique comprend un récipient de gaz contenant un mélange gazeux O₂/argon, de préférence contenant de 35 à 45% vol.% O2 et de 55 à 65% vol.% Ar.
- alternativement, la source de gaz thérapeutique comprend un premier récipient de gaz contenant de l'argon ou du N₂O, un second récipient de gaz contenant de l'oxygène (O₂) et un mélangeur de gaz alimenté en gaz par lesdits premier et second récipients de gaz, ledit mélangeur opérant un mélange des gaz provenant des premier et second récipients de gaz pour obtenir un mélange gazeux O₂/N₂O ou O₂/argon.
- le masque respiratoire est en communication fluidique avec le passage de gaz interne de l'appareil de délivrance de gaz et alimenté en gaz thérapeutique par ledit passage de gaz interne.

Dans le cadre de l'invention :
- le terme « pression » est utilisé pour désigner de façon générale, une pression positive (> 0 bar), nulle (= 0 bar) ou négative (< 0 bar), c'est-à-dire une dépression.
- les pressions sont exprimées en bar ou mbar relatifs.
- le signe « - » devant une valeur de pression signifie que la pression est négative, c'est-à-dire qu'il s'agit d'une dépression, i.e. une pression inférieure à la pression atmosphérique.
- le signe « + » devant une valeur de pression signifie que la pression est positive, i.e. une pression supérieure à la pression atmosphérique.
- les termes « gaz thérapeutique » ou « gaz respiratoire » sont considérés équivalents et désignent un gaz à un ou plusieurs constituants ou composés gazeux, c'est-à-dire un gaz 'pur' ou un mélange gazeux.
- les termes « dispositifs », « moyens », « système », « unité » ou analogues, sont considérés comme équivalents et substituables l'un à l'autre.
- les termes « contrôle », « commande », « pilotage ou similaire, sont considérés comme équivalents et substituables l'un à l'autre
- les termes « ligne », « conduite », « canalisation » et « tuyau »sont considérés comme équivalents et substituables l'un à l'autre.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de fourniture de gaz comprenant un appareil de délivrance de gaz pour fournir un gaz respiratoire à un patient.
Fig. 2 schématise un mode de réalisation d'un dispositif de raccordement et d'un module de filtration de gaz d'une installation de fourniture de gaz selon l'invention.
Fig. 3 est analogue à Fig. 2.
Fig. 4 est une vue en coupe du dispositif de raccordement de Fig. 2 et Fig. 3.

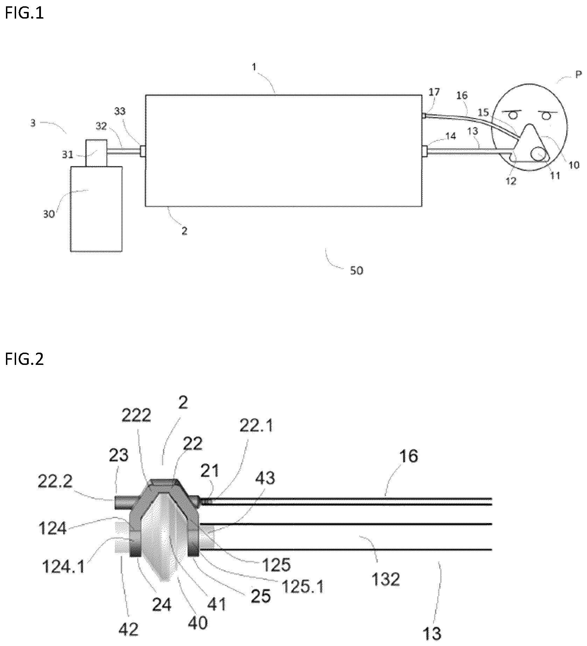

Fig. 1 est une représentation schématique d'un mode de réalisation d'une installation de fourniture de gaz 1 telle que décrite par EP3981454.

Elle comprend un appareil de délivrance de gaz 1 comprenant un boitier externe 2 formant une carcasse rigide, par exemple en polymère, comprenant les composants internes, notamment un circuit de gaz interne, un réservoir déformable, un dispositif à vanne et une unité de contrôle à microprocesseur, comme détaillé par EP3981454 auquel on peut se reporter pour plus de détail.

Une source de gaz thérapeutique 3, telle qu'une bouteille de gaz 30 équipée d'une valve 31, fournit un gaz thérapeutique, c'est-à-dire un gaz ou mélange de gaz à l'appareil de délivrance de gaz 1 via un flexible de raccordement 32, connecté au port d'entrée 33 de l'appareil de délivrance de gaz 1.

La source de gaz 3 contient un gaz thérapeutique sous pression, par exemple un mélange argon/oxygène, par exemple comprenant 60 vol.% d'argon et 40 vol.% d'oxygène, à une pression maximale de l'ordre de 250 bar. La valve 31 est de préférence un robinet à régulateur de pression intégré ou RDI délivrant le gaz dans le flexible de raccordement 32 à une pression réduite, par exemple de l'ordre de 5 bar. Le régulateur de pression intégré 31 est de préférence protégé par un chapeau rigide (non représenté).

Le gaz thérapeutique traverse l'appareil de délivrance de gaz 1, via un circuit interne d'acheminement de gaz, comme expliqué par EP3981454, pour ensuite être délivré à un patient P par le biais d'une conduite d'alimentation en gaz 13 qui est raccordée fluidiquement à un port de sortie 14 de l'appareil 1. Le gaz est fourni au patient via une interface ou masque respiratoire 10 alimenté par la conduite d'alimentation en gaz 13, typiquement un tuyau en polymère flexible, tel le circuit patient commercialisé par la société Intersurgical^{®} sous la référence 157400.

De préférence, l'interface ou masque respiratoire 10 est un masque facial, i.e. un masque naso-buccal, couvrant la bouche et le nez du patient. D'autres interfaces respiratoires pourraient bien entendu convenir.

Le masque respiratoire 10 présente un port ou un orifice d'inhalation, aussi appelé entrée de gaz 12, et un port ou un orifice d'expiration 11, aussi appelé sortie de gaz. L'entrée de gaz 12 du masque 10 est connectée fluidiquement à la conduite d'alimentation en gaz 13 qui achemine le gaz respiratoire provenant de l'appareil 1 et alimente le masque 10 avec ce gaz respiratoire.

Par ailleurs, le port d'expiration 11 permet l'évacuation à l'atmosphère des gaz lors de l'expiration du patient, c'est-à-dire des gaz expirés riches en CO₂, et empêche par ailleurs l'entrée d'air ambient dans le masque 10 lorsque le patient inhale le gaz thérapeutique, c'est-à-dire pendant ses phases inspiratoires.

Le masque 10 présente en outre un port de prise de pression, appelée sortie de pression 15, connecté fluidiquement à une ligne d'amenée de pression 16, aussi appelé « conduit d'amenée de pression », tel qu'un tuyau flexible, par exemple un tuyau en silicone, lui-même relié pneumatiquement à un capteur de pression (non représenté) agencé dans l'appareil 1, via un port de mesure de pression 17 agencé sur le boitier 2 de l'appareil de délivrance de gaz 1, de manière à pouvoir opérer des mesures de la pression régnant dans le masque 10. La ligne d'amenée de pression 16 forme donc une liaison pneumatique entre le masque respiratoire 10 et le capteur de pression interne de l'appareil de délivrance de gaz 1, permettant d'acheminer la pression gazeuse prélevée dans le masque 10 afin que le capteur de pression puisse en déterminer le niveau de pression.

L'architecture interne de l'appareil de délivrance de gaz 1 de Fig.1 et son fonctionnement étant ici en tout point identiques à ceux décrits par EP3981454, ils ne sont pas détaillés ci-après et il suffit de se reporter à ce document pour en connaître les détails.

La présente invention vise à améliorer les installations de fourniture de gaz 50 du type de celle illustrée en Fig.1 et décrite par EP3981454.

En effet, comme déjà expliqué, la longueur de la conduite d'alimentation en gaz 13 et de la ligne d'amenée de pression 16 doit être de plusieurs mètres, typiquement d'au moins 4 mètres environ, afin de prendre en compte l'encombrement fréquent des salles de soins, des chambres ou autres locaux hospitaliers.

Or, de telles longueurs de conduits de plusieurs mètres peuvent engendrer des déconnexions ou débranchements intempestifs de l'un et/ou l'autre de ces conduites ou lignes 13, 16, en particulier la conduite d'alimentation en gaz 13, lesquels ne sont pas forcément détectées immédiatement par le personnel soignant, ce qui présente alors un risque pour les patients.

Typiquement, en cas de débranchement de la conduite d'alimentation en gaz 13 au niveau de son raccordement (en 14) à l'appareil de délivrance 1 illustré en Fig. 1, alors que la ligne d'amenée de pression 16 reste elle-même correctement raccordée (en 17) à l'appareil 1, ce débranchement intempestif ne peut détecter immédiatement l'appareil 1 car la pression continue à lui être fournie par la ligne d'amenée de pression 16, ce qui incite l'appareil 1 de continuer à fonctionner (i.e. délivrer du gaz) alors qu'en réalité, le patient ne reçoit plus de gaz.

Dans le cadre de la présente invention, on vise à résoudre ce problème de non-détection de l'un ou l'autre de ces conduites ou lignes 13, 16, en particulier de la conduite d'alimentation en gaz 13.

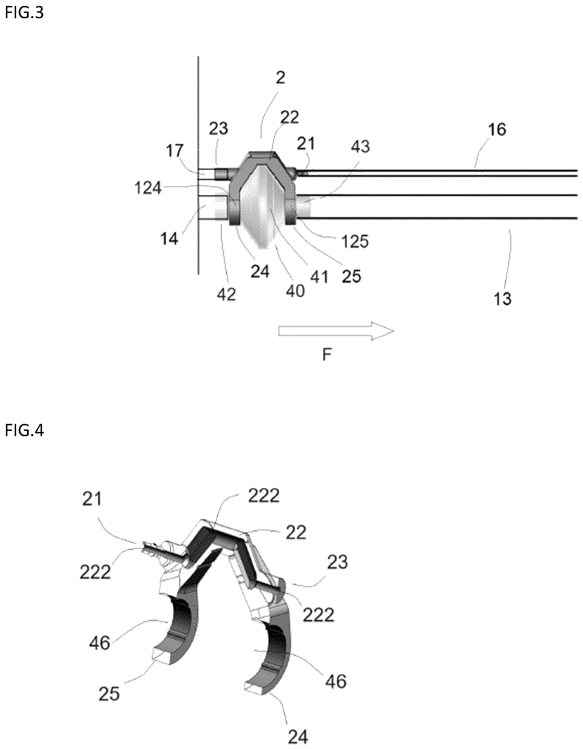

Pour ce faire, on intercale entre l'appareil 1 et les conduites ou lignes 13, 16, un ensemble 2, 40 comprenant un dispositif de raccordement 2 couplé mécaniquement à un module de filtration de gaz 40 comprenant un (des) filtre interne, typiquement un filtre bactériologique, auxquels viennent se raccorder l'appareil de délivrance de gaz 1 et, par ailleurs, la conduite d'alimentation en gaz 13 et la ligne d'amenée de pression 16, comme illustré sur Fig. 2 et Fig. 3.

Autrement dit, l'invention porte sur une installation de fourniture de gaz 50, que l'on considère, à des fins de simplification, identique à celle illustrée par exemple en Fig. 1 et décrite par EP3981454, laquelle comprend par ailleurs l'ensemble 2, 40 comprenant le dispositif de raccordement 2 et le module de filtration de gaz 40 couplés mécaniquement l'un à l'autre, comme schématisé sur Fig. 2 et Fig. 3.

**Plus précisément,** le dispositif de raccordement 2 comprend un corps principal 22 traversé par un passage interne 222, i.e. conduit, canal ou analogue, comme détaillé en Fig. 4, reliant fluidiquement la ligne d'amenée de pression 16 au port de mesure de pression 17 de l'appareil de délivrance de gaz 1 de manière à assurer une transmission de la pression mesurée au masque 10, au capteur de pression interne de l'appareil 1, qui sert à mesurer ou déterminer la pression du gaz prélevée au masque 10, amenée par la ligne d'amenée de pression 16 et entrant dans l'appareil 1 par le port de mesure de pression 17.

Le corps principal 22 du dispositif de raccordement 2 est en un matériau souple, c'est-à-dire flexible, mais robuste présentant préférentiellement une dureté Shore supérieure ou égale à 60, typiquement matériau de type silicone.

Le passage interne 222, i.e. canal ou analogue, traversant le corps principal 22 du dispositif de raccordement 2 comprend une entrée de passage 22.1 et une sortie de passage 22.2, c'est-à-dire des orifices d'entrée et de sortie communiquant fluidiquement avec le passage de gaz 222, i.e. son lumen.

Préférentiellement, le corps principal 22 comprend un connecteur amont 21, portant l'entrée de passage 22.1, configuré pour permettre un raccordement fluidique de la ligne d'amenée de pression 16 amenant la pression gazeuse prélevée dans le masque 10, et par ailleurs un connecteur aval 23, portant la sortie de passage 22.2, et configuré pour permettre un raccordement fluidique au port de mesure de pression 17 de l'appareil de délivrance de gaz 1.

Avantageusement, le connecteur amont 21 et/ou le connecteur aval 23, aussi appelés « raccords » ou analogues, ont des formes générales tronconiques ou cylindriques de manière à pouvoir être raccordés par emboitement ou similaire, à la ligne d'amenée de pression 16 et au port de mesure de pression 17, comme illustré en Fig. 3.

Par ailleurs, le dispositif de raccordement 2, typiquement le corps principal 22, comprend par ailleurs des moyens de fixation 24, 25 permettant de solidariser, c'est-à-dire de fixer le dispositif de raccordement 2 au module de filtration de gaz 131.

Dans le mode de réalisation proposé, le corps principal 22 du dispositif de raccordement 2 a une forme générale en « U » ou analogue. Il comprend deux expansions 124, 125, agencées face à face et espacées l'une de l'autre, en particulier agencées sensiblement parallèles l'une à l'autre. De préférence, elles sont symétriques l'une par rapport à l'autre.

Lorsque le corps principal 22 a, comme ici, une forme générale en « U », les deux expansions 24, 25 constituent schématiquement les deux branches verticales du « U ».

D'une façon générale, les expansions 124, 125 forment des bras, des ailes ou analogues se projetant en éloignement depuis le corps principal 22. Elles portent les moyens de fixation 24, 25. Plus précisément, les deux expansions 124, 125 comprennent chacune une extrémité proximale solidaire du corps principal et une extrémité distale libre 124.1, 125.1. Les moyens de fixation 24, 25 sont agencés au niveau des extrémités distales libres 124.1, 125.1 des expansions 124, 125.

Avantageusement, les deux expansions 124, 125 sont formées d'une seule pièce avec le corps principal 22, par exemple par injection-moulage ou analogue.

La distance entre ces deux expansions 124, 125 correspond approximativement à la largeur du corps 41 du module de filtration 40 de manière à pouvoir venir le prendre en « sandwich », comme visible sur Fig.2 et Fig. 3 et détaillé ci-après.

Plus précisément, le module de filtration 40 comprend un corps de module 41, par exemple en polymère, comprenant un compartiment ou chambre interne où est logé/agencé un (ou des) filtres, typiquement un filtre bactériologique, tel celui commercialisé par la société Intersurgical^{®} sous la référence 169000, servant à purifier le gaz qui traverse le module 40 en direction du patient P, notamment à éliminer les microorganismes pouvant s'y trouver, i.e. bactéries ou autres.

Le module de filtration 40 comprend un connecteur amont 42 et un connecteur aval 43, telles des tubulures, raccords ou analogues, agencés de part et d'autre du corps de module 41, lesdits connecteurs amont et aval 42, 43 étant en communication fluidique avec le compartiment interne du corps de module 41.

Le connecteur amont 42 est configuré pour permettre un raccordement, direct ou indirect, au port de sortie de gaz 14 de l'appareil de délivrance de gaz 1, alors que le connecteur aval 43 est configuré pour permettre un raccordement de la conduite d'alimentation en gaz 13 alimentant le masque 10, de préférence des raccordements par emboitement.

Comme on le voit sur Fig. 2 à Fig. 4, les moyens de fixation 24, 25 comprennent des ouvertures de fixation 46 (en Fig. 4) traversant la paroi des deux expansions 124, 125, au niveau des extrémités distales libres 124.1, 125.1 des expansions 124, 125, et servant à loger les connecteurs amont et aval 42, 43 du module de filtration 40 de manière à assurer une solidarisation ou un maintien du dispositif de raccordement 2, en particulier de son corps principal 22, couplé au module de filtration 40.

Etant donné que le corps principal 22 du dispositif de raccordement 2 est en matériau souple, e.g. silicone ou analogue, les connecteurs amont et aval 42, 43 du module de filtration 40 peuvent être facilement insérés dans les ouvertures de fixation 46 par déformation élastique des expansions 124, 125 du dispositif de raccordement 2. Les connecteurs amont et aval 42, 43 traversent donc les ouvertures de fixation 46.

Les connecteurs amont et aval 42, 43 du module de filtration sont sensiblement tubulaires, typiquement cylindriques, et ont par exemple une longueur de l'ordre de quelques cm.

Autrement dit, le module de filtration de gaz 40 est pris en sandwich entre les deux expansions 124, 125 du dispositif de raccordement 2 et y maintenu par les moyens de fixation 24, 24 portés par lesdites deux expansions 124, 125.

Selon l'invention, le fait d'agencer entre l'appareil de délivrance de gaz 1 et, par ailleurs, la conduite d'alimentation en gaz 13 et la ligne de mesure de pression 16, l'ensemble 2, 40 formé par le dispositif de raccordement 2 et le module de filtration de gaz 40, qui sont couplés mécaniquement l'un à l'autre, tout en étant raccordés auxdites conduites ou lignes 13, 16, n'évite pas les débranchements intempestifs qui peuvent se produire mais si une déconnexion de la conduite d'alimentation en gaz 13 du port de sortie 14 se produit, elle engendrera un débranchement simultané de la ligne d'amenée de pression 13 du port de mesure 17, ce qui interrompra la fourniture de la pression à l'appareil 1.

Dès lors, le niveau de pression dans le masque 10 ne peut plus être mesuré par le capteur de pression de l'appareil 1 et celui-ci est configuré pour déclencher une alarme sonore et/ou visuelle. A cet effet, l'unité de contrôle qui analyse la mesure de pression dans le masque 10, peut déterminer que cette pression reste sous un seuil de pression de débranchement déterminé, par exemple un seuil inférieur à 0.25 mb relatifs pendant une durée donnée, par exemple pendant 10 secondes consécutives.

Autrement dit, tout débranchement peut être immédiatement détecté et signalé à l'utilisateur, i.e. le personnel soignant, qui peut alors intervenir et opérer une reconnexion fluidique des éléments débranchés.

Le couplage du dispositif de raccordement 2 au module de filtration 40 est mieux représentée en Fig. 3 où l'on peut voir que le connecteur amont 42 (i.e. petit conduit tubulaire) du module de filtration 40 est inséré dans le port de sortie 14 de l'appareil 1, typiquement fixé par emboitement.

La rigidité mécanique conférée par les expansions 124, 125 des moyens de fixation 24, 25 du dispositif de raccordement 2, en particulier la partie terminale de l'expansion amont 124 qui est en compression sur la surface externe du corps 41 du module de filtration 40, force le connecteur aval 23 portant la sortie de passage 22.2 à venir se raccorder avec le port de mesure de pression 17, typiquement par emboitement mécanique, et réaliser alors une communication fluidique, via le passage ou canal interne 22, à la ligne d'amenée de pression 16.

En cas de traction sur le module de filtration 40, dans le sens de sa déconnexion (flèche F en Fig. 3) de l'appareil délivrance de gaz 1, par exemple du fait d'une traction intempestive opérée sur la conduite d'alimentation en gaz 13 qui est de grande longueur (>4 m env.), toute déconnexion ou déboitement du connecteur amont 42 du port de sortie 14 de l'appareil 1 s'accompagnera d'une déconnexion ou déboitement simultané du connecteur aval 23 du port de mesure de pression 17, rendant alors détectable cette déconnexion par l'appareil de délivrance de gaz 1 et avertissent alors l'utilisateur de cette déconnexion aux moyens d'alarme lesquels déclenchent alors des signaux visuels et/ou auditifs.

D'une façon générale, bien que la description précédente ait été faite, à titre purement illustratif, en lien avec l'installation de fourniture de gaz 1 décrite par EP3981454 et schématisée en Fig. 1, elle s'applique bien entendu à tout appareil de fourniture de gaz du même type, c'est-à-dire à tout appareil ou équipement de délivrance de gaz 1 équipé d'un port de sortie de gaz 14 pour fournir un gaz respiratoire à un patient via une conduit de gaz 13 alimentant une interface respiratoire de type masque 10 ou analogue, et d'un port de mesure de pression 17 permettant le raccordement d'un conduit de mesure de pression 16 relié à ladite interface respiratoire de type masque 10 afin d'y prélever la pression gazeuse qui y règne et de l'amener jusqu'à un capteur de pression interne de l'appareil 1, et où le même problème de débranchement intempestif des conduites ou lignes 13, 16 de grande longueur existe, i.e. d'au moins 4m environ.

## Revendications

1. Installation de fourniture de gaz respiratoire (50) à un patient (P) comprenant :
- un appareil de délivrance de gaz (1) comprenant un port de sortie de gaz (14) pour fournir un gaz respiratoire et un port de mesure de pression (17),
- un masque respiratoire (10) comprenant une entrée de gaz (12) et une sortie de pression (15),
- une conduite d'alimentation en gaz (13) pour acheminer le gaz respiratoire provenant du port de sortie de gaz (14) de l'appareil de délivrance de gaz (1) jusqu'à l'entrée de gaz (12) du masque (10), et
- une ligne d'amenée de pression (16) pour amener la pression de gaz de la sortie de pression (15) du masque (10) au port de mesure de pression (17) de l'appareil de délivrance de gaz (1),
**caractérisé en ce qu'**elle comporte en outre :
- un module de filtration de gaz (40) comprenant au moins un filtre, agencé entre le port de sortie de gaz (14) de l'appareil de délivrance de gaz (1) et la conduite d'alimentation en gaz (13) de sorte que le gaz provenant du port de sortie de gaz (14) traverse ledit module de filtration de gaz (40) avant d'entrer dans la conduite d'alimentation en gaz (13), et
- un dispositif de raccordement (2) comprenant un corps principal (22) traversé par un passage interne (222) mettant en communication fluidique la ligne d'amenée de pression (16) avec le port de mesure de pression (17) de l'appareil de délivrance de gaz (1), et des moyens de fixation (24, 25) pour permettre une solidarisation du dispositif de raccordement (2) au module de filtration de gaz (131).

2. Installation selon la revendication 1, **caractérisée en ce que** le corps principal (22) est en un matériau souple présentant une dureté Shore supérieure ou égale à 60.

3. Installation selon la revendication 2, **caractérisée en ce que** la conduite d'alimentation en gaz (13) et la ligne d'amenée de pression (16) ont une longueur d'au moins 4 mètres.

4. Installation selon la revendication 1, **caractérisée en ce que** le passage interne (222) du corps principal (22) comprend une entrée de passage (22.1) et une sortie de passage (22.2).

5. Installation selon la revendication 4, **caractérisée en ce que** le corps principal (22) comprend :
- un connecteur amont (21), portant l'entrée de passage (22.1), configuré pour permettre un raccordement fluidique de la ligne d'amenée de pression (16) et
- un connecteur aval (23), portant la sortie de passage (22.2), configuré pour permettre un raccordement fluidique au port de mesure de pression (17) de l'appareil de délivrance de gaz (1).

6. Installation selon l'une des revendications précédentes, **caractérisée en ce que** le corps principal (22) du dispositif de raccordement (2) comprend deux expansions (124, 125), agencées face à face et espacées l'une de l'autre, lesdites expansions (124, 125) portant les moyens de fixation (24, 25).

7. Installation selon les revendications 1 et 6, **caractérisée en ce que** le module de filtration de gaz (40) est pris en sandwich entre les deux expansions (124, 125) et maintenu par les moyens de fixation (24, 25) portés par lesdites deux expansions (124, 125).

8. Installation selon l'une des revendications précédentes, **caractérisée en ce que** le corps principal (22) du dispositif de raccordement (2) a une forme générale en U.

9. Installation selon la revendication 1, **caractérisée en ce que** l'appareil de délivrance de gaz (1) comprend un capteur de pression interne configuré pour déterminer la pression du gaz amené par la ligne d'amenée de pression (16).

10. Installation selon la revendication 1, **caractérisée en ce que** l'appareil comprend en outre des moyens d'alarme de débranchement pour alerter l'utilisateur d'une déconnexion du corps principal du dispositif de raccordement.

11. Installation selon la revendication 2, **caractérisée en ce que** le matériau souple est choisi parmi les matériaux silicone.

12. Installation selon la revendication 1, **caractérisée en ce qu'**elle comprend en outre une source de gaz thérapeutique (3) raccordée fluidiquement au circuit interne de gaz de l'appareil de délivrance de gaz (1) pour alimenter ledit passage de gaz en gaz thérapeutique.

13. Installation selon la revendication 1, **caractérisée en ce que** la source de gaz thérapeutique (3) comprend un récipient de gaz contenant un mélange gazeux O₂/N₂O.

14. Installation selon la revendication 1, **caractérisée en ce que** la source de gaz thérapeutique (3) comprend un récipient de gaz contenant un mélange gazeux O₂/argon.

15. Installation selon la revendication 1, **caractérisée en ce que** la source de gaz thérapeutique (3) comprend un premier récipient de gaz contenant de l'argon ou du N₂O, un second récipient de gaz contenant de l'oxygène (O₂) et un mélangeur de gaz alimenté en gaz par lesdits premier et second récipients de gaz, ledit mélangeur opérant un mélange des gaz provenant des premier et second récipients de gaz pour obtenir un mélange gazeux O₂/N₂O ou O₂/argon.
